# EUROPÄISCHE PATENTSCHRIFT

(11) **EP 1 221 442 B1**
(45) Veröffentlichungstag und Bekanntmachung des Hinweises auf die Patenterteilung: **28.07.2010**
(21) Anmeldenummer: 01100428.0
(22) Anmeldetag: 08.01.2001
(51) Int. Cl.: C07D 301/12

(54) **Verfahren zur Epoxidierung von Olefinen**
Epoxidation process of olefins
Procédé d' époxydation d'une oléfine

(43) Veröffentlichungstag der Anmeldung: 10.07.2002
(73) Patentinhaber: Evonik Degussa GmbH, 45128 Essen (DE); Uhde GmbH, 44141 Dortmund (DE)
(72) Erfinder: Thiele, Georg, Dr., 63450 Hanau (DE); Sauer, Jörg, Dr., Mobile, Alabama 36695 (US); Hofen, Willi, 63517 Rodenbach (DE); Haas, Thomas, Dr., 60322 Frankfurt (DE)

(56) Entgegenhaltungen:
- EP-A- 0 936 219
- US-A- 5 620 935
- US-A- 5 849 937
- US-A- 6 042 807
- US-A- 6 063 941

## Beschreibung

Die Erfindung betrifft ein Verfahren zur Epoxidierung von Olefinen mit Wasserstoffperoxid und einem Titanzeolithkatalysator.

### Stand der Technik:

Aus EP-A 100 119 ist bekannt, dass sich Propen mit Wasserstoffperoxid zu Propenoxid umsetzen lässt, wenn als Katalysator ein titanhaltiger Zeolith eingesetzt wird.

Die Katalysatoren weisen jedoch den Nachteil auf, dass sie während der Reaktion zunehmend an katalytischer Aktivität verlieren. Für die Epoxidierung von Propen ist dies in M. G. Clerici, G. Bellussi und U. Romano, J. Catal. 129 (1991) 159-167 beschrieben. Deshalb wurden Verfahren entwickelt, um durch eine periodische Regenerierung des Katalysators dessen katalytische Aktivität aufrecht zu erhalten.

WO 99/01445 betrifft ein Verfahren zur Epoxidierung von Olefinen mit Wasserstoffperoxid, bei dem durch Erhöhung von Druck und Temperatur der fortschreitenden Deaktivierung des Katalysators entgegengewirkt wird, um einen vorbestimmten Mindestumsatz so lange wie möglich aufrechtzuerhalten und somit das Intervall zwischen zwei Regenerationscyclen des Katalysators zu verlängern. Allerdings sind der Erhöhung von Druck und Temperatur technische Grenzen gesetzt. So finden nach Überschreitung einen Maximaltemperatur vermehrt Nebenreaktionen statt, so dass z. B. die Temperatur nicht beliebig gesteigert werden kann. Auch wenn der Zeitraum zwischen zwei Regenerationscyclen verlängert werden kann, ist dennoch eine getrennte Regeneration des Katalysators zwingend notwendig.

Aus Clerici et al. ist bekannt, dass der Katalysator durch Kalzinieren des Katalysators bei 550°C regeneriert werden kann. In EP-A 743 094 ist ein Verfahren zur Regenerierung durch Kalzinieren in Gegenwart von molekularem Sauerstoff bei einer Temperatur zwischen 150 und 400°C beschrieben. In EP-A 790 075 ist außerdem ein Verfahren zur Regenerierung in einem Gasstrom bei einer Temperatur von 150 bis 200°C in Abwesenheit von molekularem Sauerstoff beschrieben. Allen diesen Verfahren ist gemeinsam, dass die Regenerierung durch eine Gasphase erfolgt und in der Regel wegen der hohen erforderlichen Temperaturen der Katalysator aus dem für die Epoxidierung verwendeten Reaktor entfernt werden muss, was mit zusätzlichem Aufwand verbunden ist.

Aus Clerici et al. ist außerdem bekannt, dass der Katalysator durch Waschen mit einem Lösungsmittel bei erhöhter Temperatur regeneriert werden kann. Dieses Verfahren erfordert jedoch in der Praxis entweder sehr lange Zeiten oder wesentlich höhere Temperaturen und lässt sich deshalb in technischen Anlagen nicht wirtschaftlich einsetzen.

Aus EP-A 757 044 ist bekannt, dass sich der Katalysator durch Behandeln mit Wasserstoffperoxid in Abwesenheit eines Olefins regenerieren lässt.

DE-A 198 05 552 lehrt, dass sich dieses Verfahren auch so durchführen lässt, dass der Katalysator während der Regenerierung in dem zur Epoxidierung verwendeten Reaktor verbleibt. Das Verfahren hat jedoch den Nachteil, dass die Epoxidierungsreaktion für die Regenerierung des Katalysators unterbrochen werden muss.

In WO 98/18555 wird ein Verfahren zur Regenerierung eines Titanzeolithkatalysators, der bei der Epoxidierung von Olefinen mit Wasserstoffperoxid eingesetzt wird, beschrieben. Gemäß einer Ausführungsform wird als Regenerierungsmedium eine Lösung des Oxidationsmittels, das auch für die Epoxidierung eingesetzt wird, verwendet. Zum Beispiel kann das Reaktionsmedium nach Austritt aus dem Epoxidierungsreaktor gegebenenfalls nach Zugabe von Wasserstoffperoxid als Regenerationsmedium verwendet werden. Dieses ist dabei weitgehend frei von nicht umgesetzten Olefinen.

Aus US-A 5 849 937 ist bekannt, dass ein unterbrechungsfreier Betrieb der Epoxidierung erreicht werden kann, wenn die Umsetzung in einer Reihe von in Serie geschalteten Festbettreaktoren durchgeführt wird und bei Abnahme der Aktivität des Katalysators in einem Reaktor dieser Reaktor außer Betrieb genommen und durch einen Reaktor mit regeneriertem Katalysator ersetzt wird. Diese Vorgehensweise hat den Nachteil, dass ein Reaktor und die entsprechende Katalysatormenge mehr bereit gehalten werden müssen, als zur Durchführung der Epoxidierung erforderlich sind.

Allen bekannten Verfahren zur Regenerierung ist der Nachteil gemein, dass der Katalysator nach der Regenerierung eine sprunghaft erhöhte Aktivität aufweist und zur vermehrten Bildung von Nebenprodukten durch Folgereaktionen von Propylenoxid führt. Dies resultiert in Ausbeuteverlusten und in Problemen beim Betrieb einer kontinuierlichen Produktionsanlage durch die auftretenden Schwankungen in der Wärmeentwicklung und den Konzentrationen an Nebenprodukten.

Aufgabe der vorliegenden Erfindung ist daher, ein Verfahren zur katalytischen Epoxidierung von Olefinen mit Wasserstoffperoxid und einem Titanzeolithkatalysator zur Verfügung zu stellen, bei dem die katalytische Aktivität des Katalysators periodisch regeneriert wird, ohne dass es zu sprunghaften Änderungen durch eine nach der Regenerierung erhöhte Aktivität und Nebenproduktbildung kommt.

### Gegenstand der Erfindung:

Diese Aufgabe wird durch ein Verfahren zur katalytischen Epoxidierung von Olefinen mit Wasserstoffperoxid und einem Titanzeolithkatalysator gelöst, wobei die Epoxidierungsreaktion in einem kontinuierlich durchströmten Reaktionssystem durchgeführt wird und die Regenerierung von deaktiviertem Katalysator durch Wasserstoffperoxid in Gegenwart des Olefins bei gleichzeitiger Fortführung der Epoxidierungsreaktion erfolgt.

Es wurde überraschenderweise festgestellt, dass Wasserstoffperoxid selbst in Mischung mit dem Ausgangsolefin ohne Unterbrechung der Epoxidierungsreaktion in der Lage ist, den deaktivierten Katalysator wieder zu regenerieren. Der Erfolg der Regenerierung ist dabei um so größer, je größer die Konzentration des noch nicht umgesetzten Wasserstoffperoxids in dem Reaktionsmedium ist, das mit dem zu regenerierenden Katalysator in Kontakt tritt. Somit ist es besonders vorteilhaft, wenn der deaktivierte Katalysator zur Regeneration in dem Reaktionssystem nahe des Zulaufs von Wasserstoffperoxid angeordnet wird.

Eine besonders bevorzugte Ausführungsform der vorliegenden Erfindung betrifft ein Verfahren zur katalytischen Epoxidierung von Olefinen mit Wasserstoffperoxid in Gegenwart eines Titanzeolithkatalysator in einem kontinuierlich durchströmten Reaktionssystem, bei dem deaktivierter Katalysator aus dem Bereich des Austritts des Reaktionsproduktes aus dem Reaktionssystem nahe des Zulaufs von Wasserstoffperoxid angeordnet wird, ohne dass der Fortlauf der Reaktion unterbrochen wird.

Das erfindungsgemäße Verfahren zur Epoxidierung mit Regenerierung des Katalysators durch Wasserstoffperoxid in Gegenwart von Olefinen kann in Abhängigkeit vom gewählten Reaktionssystem auf unterschiedliche Weise ausgeführt werden.

In einer Ausführungsform der Erfindung wird die Epoxidierungsreaktion in einem Strömungsrohrreaktor durchgeführt, in dem der Katalysator in Form eines Festbetts fixiert ist und in den an einem Ende eine Mischung aus Wasserstoffperoxid, Olefin und optional einem Lösungsmittel eingespeist und am anderen Ende die Reaktionsmischung mit dem gebildeten Epoxid entnommen wird. Die erfindungsgemäße Regenerierung wird in diesem Fall durch eine Umkehrung der Strömungsrichtung erreicht, durch die der deaktivierte Katalysator vom Ende des Festbetts an die Stelle des Zulaufs von Wasserstoffperoxid gesetzt und dadurch regeneriert wird.

In einer alternativen Ausführungsform der Erfindung wird ein Reaktionssystem aus zwei oder mehr in Reihe geschalteten Reaktoren eingesetzt, in denen der Katalysator jeweils zurückgehalten wird. Dabei wird in den ersten Reaktor eine Mischung aus Wasserstoffperoxid, Olefin und optional einem Lösungsmittel eingespeist und die in diesem Reaktor gebildete Reaktionsmischung nacheinander durch die weiteren Reaktoren geleitet, wobei optional noch weiteres Olefin zwischen den Reaktoren eingespeist werden kann. In dieser Ausführungsform wird die erfindungsgemäße Regenerierung erreicht, indem die Reihenfolge der Verschaltung der Reaktoren so geändert wird, dass der Reaktor mit dem deaktivierten Katalysator als erster Reaktor in der Reihe eingesetzt wird.

In einer bevorzugten Ausführungsform wird die Reaktion in zwei oder mehr in Reihe geschalteten Festbettreaktoren durchgeführt und die Regenerierung des Katalysators so durchgeführt, dass der letzte Reaktor in der Reihe an den Beginn der Reihe gesetzt und als erster Reaktor der Reihe betreiben wird. Diese Ausführungsform kann auch realisiert werden, indem die Reihe der Festbettreaktoren als Abfolge von Reaktionszonen in einem gemeinsamen Apparat angeordnet wird und die Änderung der Reihenfolge dieser Zonen beim Durchströmen mit Reaktionsmischung durch eine geeignete Verschaltung von Zu- und Abläufen zwischen den Zonen bewerkstelligt wird.

Die Regenerierung des Katalysators kann periodisch in festen Zeitabständen erfolgen. Alternativ kann eine Kenngröße, die indikativ für die Aktivität des Katalysators ist, während des Verlaufs der Reaktion überwacht werden und bei Unterschreiten eines vorbestimmten Grenzwerts die Regenerierung des Katalysators initiiert werden. Vorzugsweise wird die Regenerierung auch in Abhängigkeit vom Wasserstoffperoxidumsatz durchgeführt, d.h. immer dann, wenn der Umsatz im Gesamtsystem oder in einem der Reaktoren unter einen vorgegebenen Wert fällt. Vorzugsweise wird die Regenerierung bei Unterschreiten eines Wasserstoffperoxidumsatzes von 90% besonders bevorzugt von 95% eingeleitet.

Alternativ kann die Katalysatoraktivität über die Wärmeentwicklung aufgrund der exothermen Epoxidierungsreaktion überwacht werden. Vorteilhafterweise kann die Temperaturdifferenz zwischen einem Messpunkt innerhalb des Reaktors oder an der Stelle des Austritts der Reaktionsmischung aus dem Reaktor und einem Messpunkt im Kühlmedium, das zur Abfuhr der Reaktionswärme aus dem Reaktor dient, gemessen werden. Der Temperaturunterschied ist annähernd proportional zur Wärmeentwicklung. Vorzugsweise wird bei dieser Ausführungsform die Regenerierung des Katalysators initiiert, wenn der Temperaturunterschied auf 20% besonders bevorzugt auf 50% der anfänglichen Temperaturdifferenz abgefallen ist. Unter anfänglicher Temperaturdifferenz ist hierbei die Temperaturdifferenz zwischen den beiden Messpunkten zu verstehen, die sich nach Erreichen des stationären Zustands im kontinuierlichen Reaktionssystems nach Anfahren des Systems mit frischem Katalysator, einstellt.

Das erfindungsgemäße Verfahren hat verschiedene Vorteile im Vergleich zum Stand der Technik. Insbesondere erfolgt bei dem erfindungsgemäßen Verfahren die Regenerierung ohne Unterbrechung der Reaktion, so dass keine Totzeiten mehr auftreten. Aus US-A 5,849,937 ist zwar ebenfalls eine Verfahren bekannt, bei dem Totzeiten vermieden werden. Hierbei ist aber ein zusätzlicher Reaktor samt Katalysatorfüllung notwendig, da zur Katalysatorregenerierung immer ein Reaktor aus dem Epoxidierungsverfahren herausgenommen wird und der Katalysator getrennt regeneriert wird. Somit fallen in Vergleich zu der Lehre von US-A 5,849,937 deutlich verringerte Investitionskosten an.

Neben diesen wirtschaftlichen Vorteilen hat das erfindungsgemäße Verfahren den Vorteil, dass kein sprunghafter Anstieg der Katalysatoraktivität nach erfolgter externer Regenerierung auftritt. Somit ist das Aktivitätsprofil der gesamten Katalysatorfüllung während des kontinuierlichen Betriebs der Anlage im Vergleich zum Stand der Technik wesentlich gleichförmiger. Dies führt zu einer gleichbleibenden Produktqualität und Schwankungen in der Wärmeentwicklung und den Konzentrationen von Nebenprodukten können deutlich minimiert werden. Das erfindungsgemäße Verfahren eignet sich zur Epoxidierung von aliphatischen, cycloaliphatischen und aliphatisch-aromatischen olefinischen Verbindungen. Bevorzugt werden Olefine mit 3 bis 8 Kohlenstoffatomen eingesetzt, besonders bevorzugt Propen und 1-Buten. Die olefinische Verbindung kann eine oder mehrere funktionelle Gruppen enthalten, wie z.B. Hydroxyl, Halogen, Alkoxy oder Carbalkoxy. Allylchlorid und Allylalkohol lassen sich im erfindungsgemäßen Verfahren gut epoxidieren.

Als Katalysator eignen sich für das erfindungsgemäße Epoxidierungsverfahren kristalline, titanhaltige Zeolithe der Zusammensetzung (TiO₂)ₓ(SiO₂)₁₋ₓ mit x von 0.001 bis 0.05. Bevorzugt werden Titanzeolithe mit einer MFI- bzw. MEL-Kristallstruktur, bekannt als Titansilicalit-1 und Titansilicalit-2, eingesetzt. Der Titanzeolithkatalysator kann als Pulver oder als verformter Katalysator in Form von Granulaten, Extrudaten oder Formkörpern eingesetzt werden. Zur Formgebung kann der Katalysator 1 bis 99 % eines Bindemittels oder Trägermaterials enthalten, wobei alle Bindemittel und Trägermaterialien geeignet sind, die unter den zur Epoxidierung angewandten Reaktionsbedingungen nicht mit Wasserstoffperoxid oder dem Epoxid reagieren. Bevorzugt werden Extrudate mit einem Durchmesser von 1 bis 5 mm eingesetzt.

Das Wasserstoffperoxid wird bei dem erfindungsgemäßen Verfahren in Form einer wässrigen Lösung mit einem Wasserstoffperoxidgehalt von 1 bis 90 Gew. -%, bevorzugt von 10 bis 70 Gew.-% und besonders bevorzugt von 30 bis 50 Gew. -% eingesetzt. Das Wasserstoffperoxid kann in Form der im Handel erhältlichen, stabilisierten Lösungen eingesetzt werden. Ebenso geeignet sind nicht stabilisierte, wässrige Wasserstoffperoxidlösungen, wie sie bei dem Anthrachinonverfahren zur Herstellung von Wasserstoffperoxid erhalten werden. Alternativ hierzu kann Wasserstoffperoxid auch als organisch-wässrige Lösung oder organische Lösung eingesetzt werden. Bevorzugt wird dem Epoxidationsreaktor eine mit einer Base versetzte und pH-kontrollierte wässrige oder wässrigorganische Wasserstoffperoxidlösung zugesetzt.

Die Reaktion wird bevorzugt in Gegenwart eines Lösungsmittels durchgeführt, um die Löslichkeit des Olefins in der wasserstoffperoxidhaltigen flüssigen Phase zu erhöhen. Als Lösungsmittel geeignet sind alle Lösungsmittel, die unter den gewählten Reaktionsbedingungen nicht oder nur in geringem Maß durch Wasserstoffperoxid oxidiert werden und sich mit mehr als 10 Gew.-% in Wasser lösen. Bevorzugt werden Lösungsmittel, die mit Wasser unbegrenzt mischbar sind. Geeignete Lösungsmittel sind Alkohole wie z.B. Methanol, Ethanol oder tert-Butanol; Glykole wie z.B. Ethylenglykol, 1,2-Propandiol oder 1,3-Propandiol; cyclische Ether wie z.B. Tetrahydrofuran, Dioxan oder Propylenoxid; Glykolether wie z.B. Ethylenglykolmonomethylether, Ethylenglykolmonoethylether, Ethylenglykolmonobutylether oder die Propylenglykolmonomethylether und Ketone wie z.B. Aceton oder 2-Butanon. Besonders bevorzugt wird Methanol als Lösungsmittel zugesetzt.

Das erfindungsgemäße Verfahren zur Epoxidierung von Olefinen wird bei einer Temperatur von -10 bis 100 °C, vorzugsweise bei 20 bis 70 °C durchgeführt. Das Olefin wird vorzugsweise im Überschuß zu Wasserstoffperoxid eingesetzt um einen weitgehenden Wasserstoffperoxidumsatz zu erreichen, wobei das molare Verhältnis von Olefin zu Wasserstoffperoxid gleich/größer 1 ist und vorzugsweise im Bereich von 1.1 bis 10 liegt. Bei Zusatz eines organischen Lösungsmittels wird die Lösungsmittelmenge vorzugsweise so gewählt, daß in der Reaktionsmischung nur eine flüssige Phase vorliegt. Bevorzugt wird das Lösungsmittel in einem Gewichtsverhältnis von 1 bis 20 relativ zur eingesetzten Wasserstoffperoxidmenge zugesetzt.

Die eingesetzte Katalysatormenge kann in weiten Grenzen variiert werden und wird vorzugsweise so gewählt, dass unter den angewandten Reaktionsbedingungen innerhalb von 1 min bis 5 h ein Wasserstoffperoxidumsatz von mehr als 90 %, vorzugsweise mehr als 95 % erreicht wird.

Wird ein Olefin umgesetzt, dessen Siedepunkt bei Normaldruck unterhalb der gewählten Reaktionstemperatur liegt, dann wird die Reaktion bevorzugt unter Druck und unter einer Atmosphäre durchgeführt, die im wesentlichen aus dem dampfförmigem Olefin besteht; ein Olefin-Partialdruck im Bereich von 0.1 bis 1 MPa ist geeignet. Der Druck wird dabei besonders bevorzugt zwischen 50 und 100 % des Sättigungsdampfdrucks des Olefins bei der Reaktionstemperatur gewählt.

## Patentansprüche

1. Verfahren zur katalytischen Epoxidierung von Olefinen mit Wasserstoffperoxid und einem Titanzeolithkatalysator, wobei die Epoxidierungsreaktion in einem kontinuierlich durchströmten Reaktionssystem durchgeführt wird und die Regenerierung von deaktiviertem Katalysator durch Wasserstoffperoxid in Gegenwart des Olefins bei gleichzeitiger Fortführung der Epoxidierungsreaktion erfolgt.

2. Verfahren nach Anspruch 2, wobei der deaktivierte Katalysator im Reaktionssystem nahe des Zulaufs von Wasserstoffperoxid angeordnet wird.

3. Verfahren nach einem der vorstehenden Ansprüche, bei dem deaktivierter Katalysator aus dem Bereich des Austritts des Reaktionsproduktes aus dem Reaktionssystem nahe des Zulaufs von Wasserstoffperoxid angeordnet wird, ohne dass der Fortlauf der Reaktion unterbrochen wird.

4. Verfahren nach einem der vorstehenden Ansprüche, bei dem die Epoxidierungsreaktion in einem Strömungsrohrreaktor durchgeführt wird, in dem der Katalysator in Form eines Festbetts fixiert ist und in den an einem Ende eine Mischung aus Wasserstoffperoxid, Olefin und gegebenenfalls einem Lösungsmittel 1 eingespeist und am anderen Ende die Reaktionsmischung mit dem gebildeten Epoxid entnommen wird und die Regenerierung durch Umkehrung der Strömungsrichtung erreicht wird, durch die der deaktivierte Katalysator vom Ende des Festbetts an die Stelle des Zulaufs von Wasserstoffperoxid gesetzt wird.

5. Verfahren nach einem der Ansprüche 1-3, in dem ein Reaktionssystem aus zwei oder mehr in Reihe geschalteten Reaktoren eingesetzt wird, in denen der Katalysator jeweils zurückgehalten wird, wobei in den ersten Reaktor eine Mischung aus Wasserstoffperoxid. Olefin und gegebenenfalls einem Lösungsmittel eingespeist und die in diesem Reaktor gebildete Reaktionsmischung nacheinander durch die weiteren Reaktoren geleitet wird und zur Regenerierung die Reihenfolge der Verschaltung der Reaktoren so geändert wird, dass der Reaktor mit dem deaktivierten Katalysator als erster Reaktor in der Reihe eingesetzt wird.

6. Verfahren nach einem der Ansprüche 1-3, bei dem die Reaktion in zwei oder mehr in Reihe geschalteten Festbettreaktoren durchgeführt wird, in die an einem Ende eine Mischung aus Wasserstoffperoxid, Olefin und gegebenenfalls einem Lösungsmittel eingespeist und am anderen Ende die Reaktionsmischung mit dem gebildeten Epoxid entnommen wird und zur Regenerierung des deaktivierten Katalysators der letzte Reaktor in der Reihe an den Beginn der Reihe gesetzt und als erster Reaktor der Reihe betreiben wird.

7. Verfahren nach einem der Ansprüche 1-3, bei dem der Katalysator in einer Abfolge von in Reihe geschalteten Reaktionszonen in einem gemeinsamen Apparat angeordnet wird, in den an einem Ende eine Mischung aus Wasserstoffperoxid. Olefin und gegebenenfalls einem Lösungsmittel eingespeist und am anderen Ende die Reaktionsmischung mit dem gebildeten Epoxid entnommen wird und die Reihenfolge dieser Zonen beim Durchströmen mit Reaktionsmischung durch eine geeignete Verschaltung von Zu- und Abläufen zwischen den Zonen so geändert wird, dass die Zone mit dem deaktivierten Katalysator als erste Reaktionszone durchströmt wird.

8. Verfahren nach einem der Ansprüche 5-7, bei dem weiteres Olefin zwischen den Reaktoren bzw. Reaktionszonen eingespeist wird.

9. Verfahren nach einem der vorstehenden Ansprüche, in dem die Regenerierung des Katalysators periodisch in festen Zeitabständen erfolgt.

10. Verfahren nach einem der Ansprüche 1-8, in dem eine Kenngröße, die indikativ für die Aktivität des Katalysators ist, während des Verlaufs der Reaktion überwacht wird und bei Unterschreiten eines vorbestimmten Grenzwerts die Regenerierung des Katalysators initiiert wird.

11. Verfahren nach Anspruch 10, bei dem die Kenngröße ausgewählt ist aus Wasserstoffperoxidumsatz und Wärmeentwicklung.

## Claims

1. Process for the catalytic epoxidation of olefins by means of hydrogen peroxide and a titanium zeolite catalyst, wherein the epoxidation reaction is carried out in a reaction system through which the reaction mixture flows continuously and the regeneration of deactivated catalyst is carried out by means of hydrogen peroxide in the presence of the olefin while continuing the epoxidation reaction.

2. Process according to Claim 2, wherein the deactivated catalyst is located close to the point at which hydrogen peroxide enters the reaction system.

3. Process according to any of the preceding claims, wherein deactivated catalyst from the region in which the reaction product leaves the reaction system is located close to the point at which hydrogen peroxide enters, without the reaction being interrupted.

4. Process according to any of the preceding claims, wherein the epoxidation reaction is carried out in a flow tube reactor in which the catalyst is fixed in the form of a fixed bed and in which a mixture of hydrogen peroxide, olefin and optionally a solvent is fed in at one end and the reaction mixture comprising the epoxide formed is taken off at the other end and the regeneration is achieved by reversal of the flow direction so that the deactivated catalyst is transposed from the end of the fixed bed to the point at which hydrogen peroxide enters.

5. Process according to any of Claims 1-3, wherein use is made of a reaction system comprising two or more reactors connected in series in which the catalyst is in each case retained, where a mixture of hydrogen peroxide, olefin and optionally a solvent is fed into the first reactor and the reaction mixture formed in this reaction is passed successively through the further reactors and regeneration is achieved by changing the order in which the reactors are connected so that the reactor containing the deactivated catalyst becomes the first reactor in the series.

6. Process according to any of Claims 1-3, wherein the reaction is carried out in two or more fixed-bed reactors connected in series into which a mixture of hydrogen peroxide, olefin and optionally a solvent is fed at one end and the reaction mixture comprising the epoxide formed is taken off at the other end and regeneration of the deactivated catalyst is achieved by placing the last reactor in the series at the beginning of the series and operating it as first reactor of the series.

7. Process according to any of Claims 1-3, wherein the catalyst is located in a sequence of reaction zones connected in series in a common apparatus into which a mixture of hydrogen peroxide, olefin and optionally a solvent is fed at one end and the reaction mixture comprising the epoxide formed is taken off at the other end and the order in which the reaction mixture flows through these zones is changed by appropriate switching of inlets and outlets between the zones so that the reaction mixture flows through the zone containing the deactivated catalyst as first reaction zone.

8. Process according to any of Claims 5-7, wherein further olefin is fed in at points between the reactors or reaction zones.

9. Process according to any of the preceding claims, wherein the regeneration of the catalyst is carried out periodically at fixed time intervals.

10. Process according to any of Claims 1-8, wherein a parameter which is indicative of the activity of the catalyst is monitored during the reaction and the regeneration of the catalyst is initiated when the activity drops below a predetermined threshold value.

11. Process according to Claim 10, wherein the parameter is selected from among hydrogen peroxide conversion and evolution of heat.

## Revendications

1. Procédé pour l'époxydation catalytique d'oléfines avec du peroxyde d'hydrogène et un catalyseur zéolithique de titane, où la réaction d'époxydation est réalisée dans un système de réaction traversé en continu et la régénération du catalyseur désactivé est réalisée par du peroxyde d'hydrogène en présence de l'oléfine en poursuivant simultanément la réaction d'époxydation.

2. Procédé selon la revendication 2, où le catalyseur désactivé est disposé dans le système de réaction à proximité de l'alimentation du peroxyde d'hydrogène.

3. Procédé selon l'une quelconque des revendications précédentes, dans lequel le catalyseur désactivé de la zone de la sortie du produit de réaction hors du système de réaction est disposé à proximité de l'alimentation du peroxyde d'hydrogène sans que la poursuite de la réaction ne soit interrompue.

4. Procédé selon l'une quelconque des revendications précédentes, dans lequel la réaction d'époxydation est réalisée dans un réacteur tubulaire à écoulement, dans lequel le catalyseur est fixé sous forme d'un lit fixe et dans lequel on injecte en une extrémité un mélange de peroxyde d'hydrogène, d'oléfine et le cas échéant d'un solvant et on prélève en l'autre extrémité le mélange réactionnel avec l'époxyde formé et la régénération est réalisée par l'inversion du sens d'écoulement par laquelle le catalyseur désactivé de l'extrémité du lit fixe est placé au site d'alimentation du peroxyde d'hydrogène.

5. Procédé selon l'une quelconque des revendications 1-3, dans lequel on utilise un système de réaction constitué par deux réacteurs commutés en série ou plus, dans lesquels le catalyseur est à chaque fois retenu, où on injecte dans le premier réacteur un mélange de peroxyde d'hydrogène, d'oléfine et le cas échéant d'un solvant et le mélange réactionnel formé dans ce réacteur est guidé consécutivement au travers des autres réacteurs et on modifie, pour la régénération, l'ordre de la commutation des réacteurs de manière telle que le réacteur avec le catalyseur désactivé est utilisé comme premier réacteur dans la série.

6. Procédé selon l'une quelconque des revendications 1-3, dans lequel la réaction est réalisée dans deux réacteurs à lit fixe commutés en série ou plus, dans lesquels on injecte en une extrémité un mélange de peroxyde d'hydrogène, d'oléfine et le cas échéant d'un solvant et on prélève en l'autre extrémité le mélange réactionnel avec l'époxyde formé et on place, pour la régénération du catalyseur désactivé, le dernier réacteur dans la série au début de la série et on l'exploite comme premier réacteur de la série.

7. Procédé selon l'une quelconque des revendications 1-3, dans lequel le catalyseur est disposé dans une succession de zones de réaction disposées en série dans un appareil commun, dans lequel on injecte en une extrémité un mélange de peroxyde d'hydrogène, d'oléfine et le cas échéant d'un solvant et on prélève en l'autre extrémité le mélange réactionnel avec l'époxyde formé et la succession de ces zones est modifiée, lors du passage du mélange réactionnel par une commutation appropriée des alimentations et évacuations entre les zones de manière telle que la zone contenant le catalyseur désactivé est traversée en tant que première zone de réaction.

8. Procédé selon l'une quelconque des revendications 5-7, dans lequel de l'oléfine supplémentaire est injectée entre les réacteurs ou les zones de réaction.

9. Procédé selon l'une quelconque des revendications précédentes, dans lequel la régénération du catalyseur est réalisée périodiquement, à des intervalles de temps fixes.

10. Procédé selon l'une quelconque des revendications 1-8, dans lequel une grandeur caractéristique, indicatrice de l'activité du catalyseur, est suivie pendant le déroulement de la réaction et la régénération du catalyseur est initiée lors du dépassement au-dessous d'une valeur limite prédéfinie.

11. Procédé selon la revendication 10, dans lequel la grandeur caractéristique est choisie parmi la conversion du peroxyde d'hydrogène et le dégagement de chaleur.
